(19) Europäisches Patentamt
European Patent Office
Office européen des brevets

(11) Publication number: **0 250 181 A2**

(12) # EUROPEAN PATENT APPLICATION

(21) Application number: 87305251.8

(22) Date of filing: 12.06.87

(51) Int. Cl.[4]: C11D 1/94 , A61K 7/08

(30) Priority: 16.06.86 US 874554
12.05.87 US 47073

(43) Date of publication of application:
23.12.87 Bulletin 87/52

(84) Designated Contracting States:
AT BE CH DE ES FR GB IT LI LU NL SE

(71) Applicant: Helene Curtis Industries, Inc.
325 North Wells Street
Chicago Illinois 60601(US)

(72) Inventor: LaPetina, Donna M.
2 S 030 Bristol Ln.
Warrenville Illinois 60555(US)
Inventor: Patel, Chaitanya
1118-1A Prescott
Roselle Illinois 60172(US)

(74) Representative: Wain, Christopher Paul et al
A.A. THORNTON & CO. Northumberland
House 303-306 High Holborn
London WC1V 7LE(GB)

(54) **Mild detergent compositions.**

(57) A mild detergent composition, including a carboxylated derivative of a fatty imidazoline and a zwitterionic betaine or sultaine together with a sulfated alkylphenol ethoxylate, unexpectedly provides the unusually low skin and eye irritation levels associated with baby shampoos while exhibiting the cleansing, foaming and aesthetic properties associated with conventional shampoos.



Xerox Copy Centre

# MILD DETERGENT COMPOSITIONS

The present invention relates to very mild and non-irritating detergent and shampoo compositions having a minimal tendency to sting the eyes. More particularly, the present invention is directed to hair shampoo compositions possessing low ocular and dermal irritation potential and yet exhibiting good foam volume and stability, and improved product aesthetics. The composition of the present invention, comprising a carboxylated derivative of a fatty imidazoline, a zwitterionic betaine or sultaine and a sulfated alkylphenol ethoxylate provides a hair shampoo ideally suited for use on infants and babies by unexpectedly exhibiting improved low levels of eye and skin irritation, plus improved cleansing and aesthetic characteristics.

Conventional hair shampoos generally contain an effective amount of an anionic surface active agent, usually an alkyl sulfate or an alkyl ether sulfate, in conjunction with nonionic or amphoteric surfactants and other common additives to provide a shampoo composition that effectively cleans the hair, generates copious and stable foam, and possesses the aesthetic qualities, such as viscosity, conditioning effects, appearance and feel that make the composition acceptable to the consuming public. For baby shampoo compositions, the most important property is an extremely low level of dermal and ocular irritation and, particularly, lack of eye sting upon contact.

To achieve the low irritation levels and extreme mildness required of baby shampoo compositions, the baby shampoo is formulated from the least irritating surfactants available. The anionic surfactants commonly used in conventional hair shampoos exhibit superior foaming and cleaning properties, but they are somewhat irritating to the skin and particularly irritating to the eyes. This high irritation potential makes these anionic surfactants unsuitable for use in baby hair shampoo compositions, unless their high irritation potential can be modified and reduced.

Accordingly, baby shampoos usually contain substantial amounts of the milder and less irritating nonionic, amphoteric and zwitterionic surface active agents. However, the benefits of improved mildness and reduced irritation potential in baby shampoo compositions gained by using these surfactant-types is offset by inferior foaming and lathering, poor viscosity and oftentimes poorer cleaning compared to anionic surfactant-based shampoos. Therefore, members of the mild surfactant-based shampoos. Therefore, members of the mild surfactant groups are often combined with the harsher anionic surfactants to produce a shampoo with good cleaning capabilities and good aesthetic qualities, but with eye irritation levels still too high for baby shampoos. These results have led to extensive research efforts to find baby shampoo compositions that are mild and non-irritating to the skin and eyes; are capable of satisfactory foaming, lathering and cleaning; and possess the requisite aesthetic qualities, such as viscosity, for consumer appeal.

It is particularly desirable that shampoo compositions have good foam volume and foam stability. The amount of foam generated by a shampoo composition has a direct bearing on the perceived efficiency of cleaning the hair, and the stability of the generated foam determines how long the shampoo will keep the hair lathered. Generally speaking, the greater the volume of foam produced and the more stable the foam, the more efficient the perceived cleansing action of the shampoo. In addition, a baby shampoo composition must be extremely mild and possess low ocular and dermal irritation levels.

Non-irritating shampoo and detergent compositions have been known and in use for some time. U.S. Patent Nos. 2,999,069 and 3,055,836 are representative of such prior art non-irritating compositions, and generally comprise an amphoteric surfactant combined with an anionic surfactant and a nonionic surfactant. These prior art compositions, although non-irritating, possess foaming characteristics that are perceptively inferior in foam volume and foam stability when compared to conventional shampoos. These prior art shampoos also have very low viscosities, a characteristic still prevalent in present day non-irritating shampoos. Increasing the viscosity of these compositions by employing viscosity building additives or thickeners is counterproductive since the foaming characteristics of the compositions are further reduced. Therefore, increased viscosity, without sacrificing foaming properties, is highly desirable in formulating non-irritating shampoo compositions intended for mass marketing.

Some mild shampoo compositions have been formulated from mixtures of anionic, zwitterionic, amphoteric, ampholytic and nonionic surfactants. In these compositions, the nonionic and amphoteric surfactants have been included to modify the harshness of the anionic surfactants. All of the compositions include nonionic and anionic surfactants as essential components although the combination of both nonionic and anionic surfactants have disadvantages when incorporated into non-irritating shampoos. For examples of such compositions, see U.S. Patent Nos, 3,928,251; 3,950,417; 3,962,418; 4,177,171; 4,263,178; 4,426,310 and 4,435,300. Other disclosures considered relevant are British patent specification Nos. 1,508,929, 1,540,301 (like U.S. Patent No. 4,177,171) and 2,026,532, and French Patent No. 1,403,213. As

shown in the prior art, to produce a shampoo composition with low eye irritation, more is required than merely admixing any number of different surfactant types. Although numerous cleansing and shampoo compositions are available commercially, there remains a great demand for compositions with low irritation potential that have not sacrificed other valuable features such as cleansing, foaming and viscosity. As also known in the shampoo art, particular types of surfactants are employed to produce an acceptable irritation-free shampoo composition that will not sting an infant's eyes should accidental contact occur.

The present invention is directed to a mild detergent composition that possesses the non-irritating and non-sting properties of a baby shampoo, while retaining the cleaning and aesthetic properties of a conventional shampoo while maintaining unexpectedly better foaming properties for a baby shampoo. The detergent composition of the present invention includes components known individually in the shampoo art, but that heretofore have not been combined to produce a shampoo composition exhibiting such new and unexpected improvements in mildness and irritation, while unexpectedly maintaining cleaning and aesthetic characteristics at levels previously found only in harsher conventional shampoos.

The hair shampoo composition has the characteristics of improved mildness and reduced irritation potential over present day commercial baby shampoo compositions, while achieving new and unexpected lathering and shampoo properties. The shampoo composition of the present invention includes unique blend of anionic and amphoteric surfactants to provide the aesthetic and cleansing capabilities found in conventional shampoos while achieving improvements upon the dermal and ocular irritation levels of present day commercial baby shampoos.

In brief, it has been found that a mixture of a carboxylated derivative of a fatty imidazoline, a zwitterionic betaine or sultaine and a sulfated alkylphenol ethoxylate in a liquid carrier provides a hair shampoo composition with unexpectedly low dermal and ocular irritancy, while achieving the cleansing and aesthetic characteristics of harsher, conventional anionic surfactant-based shampoos.

Therefore, it is an object of the present invention to provide improved shampoo compositions.

Another object of the present invention is to provide improved shampoo compositions exhibiting low dermal and ocular irritation potential.

Another object of the present invention is to provide improved shampoo compositions exhibiting good foaming properties, including excellent foam stability, in addition to exhibiting low dermal and ocular irritation potential.

Another object of the present invention is to provide improved shampoo compositions exhibiting excellent aesthetic characteristics in addition to low dermal and ocular irritation potential and good foaming properties.

Another object of the present invention is to provide a liquid hair shampoo composition that effectively cleanses the hair, generates copious amounts of stable foam, and virtually will not irritate the skin or eyes.

Another object of the present invention is to provide shampoo compositions that are sufficiently non-irritating and non-stinging for use in baby shampoos while possessing the cleansing and aesthetic characteristics associated with conventional shampoos.

Another object of the present invention is to provide a liquid hair shampoo composition containing a combination of two amphoteric surfactants and an anionic surfactant to achieve unexpectedly low ocular and dermal irritation potential, high viscosity and good foaming characteristics.

According to the present invention there is provided a mild detergent composition characterised in that it comprises about 2% to about 10% by weight, based on the total weight of the composition, of a water soluble anionic surfactant of the formula

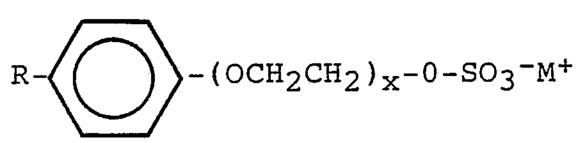

wherein R is an alkyl group containing about 6 to about 15 carbon atoms, x is an integer from 1 to about 10, and M is an alkali metal, ammonium, triethanolammonium, monoethanolammonium, diethanolammonium, or an alkylammonium containing 1 to 3 alkyl groups, each having 1 or 2 carbon atoms; about 3% to about 10% by weight, based on the total weight of the composition of an amphoteric, water soluble, carboxylated derivative of a fatty imidazoline; and about 1% to about 6% by weight, based on the total weight of the composition, of a water soluble betaine-type detergent of the formula

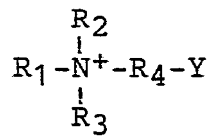

wherein $R_1$ is an alkyl or acyl group containing about 8 to about 18 carbon atoms, $R_2$ and $R_3$, the same or different, are each an alkyl group having 1 to 4 carbon groups or 2-hydroxyethyl; Y is a carboxylate or sulfonate radical, and $R_4$ is a methylene group when Y is a carboxylate group or propylene or 2-hydroxypropylene when Y is a sulfonate group; in a suitable carrier.

The invention will now be described in more detail.

The hair shampoo composition of the present invention comprises a blend of amphoteric and anionic surfactants, exhibiting unexpected mildness and achieving the cleansing and aesthetic qualities not normally found in mild detergents. The unique blend of surfactants provides a novel hair shampoo composition possessing low ocular and dermal irritation potential, making the compositions of the present invention ideal as baby shampoos.

Non-irritating detergent compositions are generally comprised of amphoteric surfactants combined with anionic and/or nonionic surfactants. The anionic surfactant is usually an ethoxylated alkyl sulfate such as ammonium lauryl ether sulfate, and the nonionic surfactant is an ethoxylated monoester of an aliphatic polyhydric alcohol. Recently, other surfactants have been included to improve the aesthetic properties of the detergent composition without increasing the irritation potential. Among these surfactants are zwitterionic betaine compounds, nonionic amine oxides, and carboxylated complexes. However, the prior art does not disclose a detergent composition containing traditional amphoteric surfactants in combination with an anionic salt of a sulfated alkylphenol ethoxylate.

The blend of surfactants included in the detergent compositions of the present invention provides extremely and unexpectedly low ocular irritation making the detergent compositions especially suitable for use as a baby shampoo composition. Additionally, the aesthetic properties of the detergent compositions are excellent. The detergent compositions or shampoos of the present invention can be formulated with Brookfield viscosities well above 1000 cps, whereas the presently available commercial products do not possess viscosities over about 1500 cps level. The foaming properties of the blend of surfactants incorporated in the detergent compositions of the present invention are excellent as well, as demonstrated by hair clinic results described in more detail hereinafter.

The non-irritating detergent compositions comprise a blend of an anionic detergent and two amphoteric detergents to obtain a hair shampoo composition of very low irritation potential while retaining high foaming and suitable viscosity characteristics heretofore known only in harsher conventional, highly anionic compositions. The total amount of the active surfactant compounds, including the nonionic surfactants added for feel or viscosity enhancement, should not be greater than about 35% by weight of the total composition in order to prevent ocular irritation. Preferably, the total amount of the active surfactant compounds will range from about 8% to about 15% by weight of the total shampoo composition.

The detergent compositions include an anionic surfactant comprising a salt of a sulfated alkylphenol ethoxylate, generally depicted by the structural formula I:

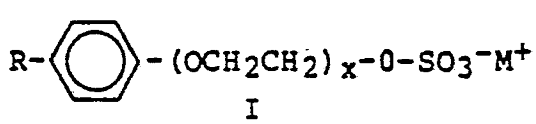

I

wherein R is an alkyl group of about 6 to about 15 carbon atoms, x is an integer from 1 to about 10, and M is sodium, potassium, ammonium or a mono-di-, or tri-alkanolamine. The compounds of structure I are also known as salts of alkylphenoxypoly(ethyleneoxy)ethanol sulfates. To achieve the full advantage of the present invention, the anionic surfactant of the detergent composition includes an alkyl group of about 7 to about 10 carbon atoms (R = 7 to 10) and contains about 4 ethyleneoxy moieties per molecule (x = 4). To achieve the fullest advantage of the present invention the anionic surfactant of the present invention is the ammonium salt of nonylphenoxypoly(ethyleneoxy)ethanol sulfate containing about 4 moles of ethylene oxide, having the CFTA adopted name of ammonium nonoxynol-4 sulfate.

The anionic surfactants of formula I provide several advantages over other anionic surfactants commonly used in hair shampoo compositions. Most notably, ammonium nonoxynol-4 sulfate has a lower primary skin irritation level than other high foaming anionics, mild surfactants or foam boosters such as alpha olefin sulfonates, lauryl sulfates, lauryl ether sulfates and cocamidopropyl betaines. Due to this inherent mildness, the ammonium nonoxynol-4 sulfate may be blended with amphoterics to provide a mild, non-stinging shampoo without the need to add large amounts of a nonionic surfactant.

The anionic surfactants of structural formula I should be present in the hair shampoo composition from about 2% to about 10% by weight, and preferably from about 2% to about 7% by weight. At this level, compositions formulated with anionics of structural formula I exhibit definite viscosity improvement, improved hard water foaming heights over sodium or ammonium lauryl sulfate detergents, very good lathering, and improved softness, luster, body and manageability of the hair. Hair shampoos formulated with anionics such as ammonium nonoxynol-4 sulfate also retain their excellent foaming levels in the presence of substantial levels of emollients.

The hair shampoo compositions include two amphoteric surfactants of different chemical classes. One of the amphoterics is a carboxylated derivative of a fatty imidazoline generally depicted by structural formula II or by structural formula III:

$$\begin{array}{l} \quad\;\; O \qquad\qquad\qquad\quad CH_2CO_2^- \\ \quad\;\; \| \qquad\qquad\qquad\qquad\;\; | \\ R-C-NHCH_2CH_2-{}^{+}N-CH_2CO_2H \qquad (Zwitterion) \\ \qquad\qquad\qquad\qquad\qquad\;\; | \\ \qquad\qquad\qquad\qquad\qquad CH_2CH_2OH \end{array}$$

II

wherein R is an alkyl radical containing predominantly 7 to 17 carbon atoms. The zwitterions of structure II are termed alkoamphocarboxyglycinates, and they exhibit low skin and eye irritation potential and also act to detoxify other composition ingredients, making them less irritating. Overall, these zwitterions demonstrate mildness, mild foaming and cleansing, and excellent performance in the presence of oily soils, making them particularly useful in baby shampoo compositions. To achieve the full advantage of the present invention, a compound of structural formula II, wherein R is the alkyl group of coconut fatty acid, cocoamphocarboxyglycinate, is present in the shampoo compositions, in an amount of about 3% to about 10% by weight and particularly in an amount of about 4% to about 7% by weight of the total composition. Representative commercial products providing the alkoamphocarboxyglycinates of structural formula II include those sold under the trade names of MIRANOL C2M CONC. N.P., MIRANOL C2M CONC. 0.P., MIRANOL CM SPECIAL (Miranol Inc.); ALKATERIC 2CIB (Alkaril Chemicals); AMPHOTERGE W-2 (Lonza Inc.); MONATERIC CDX-38, MONATERIC CSH-32 (Mona Industries); REWOTERIC AM-2C (Rewo Chemical Group); and SCHERCOTERIC MS-2 (Scher Chemicals).

Similarly the amphoteric generally depicted by structural formula III:

$$\begin{array}{l} \quad\;\; O \qquad\qquad\qquad\qquad\quad CH_2CH_2OCH_2CH_2CO_2^- \; Na^+ \\ \quad\;\; \| \qquad\qquad\qquad\qquad\quad\;\; | \\ R-C-NH-CH_2CH_2-N-CH_2CH_2CO_2^- \; Na^+ \end{array}$$

III

wherein R is an alkyl radical containing predominantly 7 to 17 carbon atoms can be used on the composition of the present invention as the carboxylated derivative of a fatty imidazoline. The zwitterions of structure III are termed alkoamphocarboxypropionates, and they likewise exhibit low skin and eye irritation potential and act to detoxify other composition ingredients, making them less irritating. The amphoterics depicted by structural formula III also demonstrate the mildness, mild foaming and cleansing and excellent performance in the presence of oily soils, making them particularly useful in baby shampoo compositions. To achieve the full advantage of the present invention, either the cocoamphocarboxyglycinate described above, or the compound having the structural formula III, wherein R is the alkyl group of coconut fatty acid, cocoamplocarboxypropionate, is present in the shampoo compositions, in an amount of about 3% to about 10% by weight and particularly in an amount of about 4% to about 7% by weight of the total composition.

Representative commercial products providing the cocoamphocarboxypropionate of structural formula III include those sold under the trade names of MIRANOL C2M-SF 70%; MIRANOL C2M-SF 75%; MIRANOL C2M-SF CONC., MIRANOL C2M-SFE CONC., MIRANOL C2M-SFP (Miranol Inc.); AMPHOTERGE K-2 (Lonza Inc.); CYCLOTERIC DC-SF (Cyclo Corp.); MONATERIC 805-P2 (Mona Industries); REWOTERIC AM-2CSF (Rewo Chemical Group); and SCHERCOTERIC MS-SF-2 (Scher Chemicals).

The hair shampoo composition contains a second amphoteric surfactant classified as a betaine or betaine-type surfactant falling within structural formulae IV through VII:

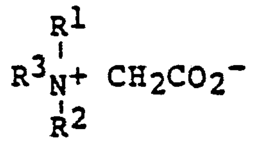 (betaine)

IV

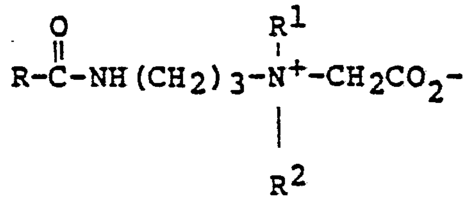 (alkamidopropyl betaine)

V

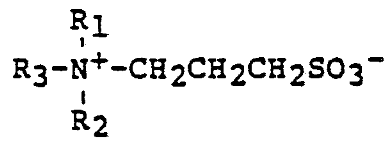 (sulfobetaine)

VI

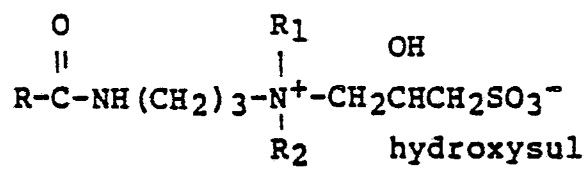 (alkamidopropyl hydroxysultaine)

VII

wherein R is an alkyl radical containing predominantly 7 to 17 carbon atoms, $R_1$ and $R_2$ are the same or different alkyl radicals having 1 to 4 carbon atoms, and $R_3$ is an alkyl radical containing predominantly 8 to 18 carbon atoms.

Zwitterionic surfactants of this class have properties advantageous to shampoos, including mildness; compatibility with a variety of different components; efficacy over a broad pH range; good foam generation, foam boosting and foam stabilizing; good detergency and viscosity enhancement. In hair shampoo compositions, particularly at an acid pH, the betaine class of surfactants also impart conditioning and antistatic properties to the hair. Each of the four types of betaine or sulfobetaine surfactants defined by structural formulae IV through VII may be used in the present invention. To achieve the full advantage of the present invention, the alkamidopropyl betaines (V) and alkamidopropyl hydroxysultaines (VII) are preferred in that they are milder, impart better conditioning properties and generate greater foam volume than the other betaine-type surfactants.

The betaines have demonstrated excellent compatibility with the mucous membranes, therefore, by themselves, they are non-irritating to the eyes. Betaines also are effective in significantly reducing the eye irritation potential of alkyl sulfates and alkyl ether sulfates. Shampoos or detergents containing only anionic surfactants will defat the skin and hair after prolonged use resulting in the formation of dandruff; however, combining an anionic surfactant with a betaine-type surfactant inhibits the defatting tendency and resultant dandruff formation. Blending a betaine-type surfactant and an anionic surfactant reduces the harshness of the anionic surfactant. In accordance with the principles of the present invention, the combination of a amphoteric surfactant together with the anionic-betaine blend heretofore defined, results in a surfactant mixture that is non-irritating and free of sting. To achieve the full advantage of the present invention, a cocamidopropyl betaine or a cocamidopropyl hydroxysultaine is incorporated into the hair shampoo composition of the present invention from levels of about 1% to about 6% by weight based on the total weight of the composition. More preferably, the cocamidopropyl betaine or cocoamidopropyl hydroxy sultaine is present in an amount of about 2% to about 4% by weight based on the total weight of the composition.

The hair shampoo compositions contain a blend of an anionic surfactant and two amphoteric surfactants to produce a non-irritating, no-sting shampoo; however, unlike other non-irritating, no-sting shampoos, the hair shampoo compositions of the present invention do not require relatively large amounts of a nonionic surfactant to eliminate the stinging characteristics of the anionic/amphoteric blend. Since nonionic surfactants are low to moderate foamers, they often detract from the excellent foaming characteristics of the anionic/amphoteric surfactant blend. By obtaining a "no-sting" quality without large percentages of a nonionic surfactant, the foaming and lather capabilities of the compositions of the present invention are superior to baby shampoo compositions requiring large amounts of nonionic surfacts to reduce sting. This will become more apparent from the performance evaluation studies discussed in more detail hereinafter.

The mild liquid detergent compositions of the present invention are essentially unbuilt liquids, i.e., do not contain amounts of organic or inorganic salts in detergent building proportions, and are therefore suitable for use as shampoos or liquid bubble bath or liquid soap products. In accordance with the present invention, traditional amphoterics, well known in the hair shampoo art, are included in the hair shampoo compositions, together with an alkylphenoxypoly(ethyleneoxy)ethanol · sulfate instead of the traditional anionic surfactant, an ethoxylated alkyl sulfate to provide hair shampoo compositions of unexpectedly improved mildness over present day commercial baby shampoos, while retaining the superior aesthetic, cleansing and conditioning characteristics normally found only in the harsher, strongly anionic traditional shampoos.

In preparing the detergent compositions of the present invention, various other surfactants, such as anionics, nonionics and the like, can be utilized, if desired, as long as the basic properties of the hair shampoo are not adversely affected. The compositions of the present invention may contain the following types of components for enhanced aesthetic properties such as foaming, feel of lathering, thickening or conditioning: amine oxides, a nonionic surfactant for feel, foaming and conditioning; sulfosuccinates, an anionic surfactant for foam boosting; alkyl ($C_8$-$C_{10}$) glucosides, a very mild nonionic surfactant for foam boosting and foam stability, for detergency, and for feel; polyethylene glycol esters for viscosity enhancement; and/or glucose esters for conditioning and viscosity enhancement.

The pH of the hair shampoo compositions of the present invention should be in the range of about 5.5 to about 7.5, and to achieve the full advantage of the present invention the pH of the composition should be between about 6.0 and about 7.0.

Other common additives may be incorporated into the compositions of the present invention, as long as the basic properties of the hair shampoo, especially with respect to eye and skin irritation potential, are not substantially adversely affected. These additives include, but are not limited to, commonly used and non-irritating dyes, fragrances, opaci ficiers, pearlescing agents, buffering agents, pH adjusters, preservatives, emollients, sequestering agents, and the like, and will usually be present in weight percentages of less than 1% each, and 2% to 5% in total. The cosmetic carrier is generally water, since the addition of most organic solvents to the compositions will increase the irritation potential of the compositions, making them unsuitable for use on babies and infants. The compositions of the present invention have sufficient inherent viscosity making it unnecessary to add organic thickeners, such as gums, or inorganic salts, such as sodium chloride or ammonium chloride.

A mild, non-stinging hair shampoo composition, suitable for use on infants and babies, is prepared by admixing a sulfated alkylphenol ethoxylate and a cocoamphocarboxyglycinate or a cocoamphocarboxypropionate with an alkamidopropyl betaine or an alkamidopropyl hydroxysultaine to yield a composition of unexpectedly low irritation potential, yet having the aesthetic and cleansing properties normally associated only with harsher, traditional anionic surfactant-based shampoos.

Test solutions were prepared to determine the eye irritation levels and the lathering and performance levels of shampoos of the present invention and present day commercial shampoos. The detergent compositions can be tested for ocular irritation by the following modified Draize Test (J.H. et al., Toilet Goods Assn. No. 17, May 1952, No. 1, Proc. Sci. Sect.).

An 0.1 ml. sample of a neutral composition under test is dropped into one eye of an albino rabbit, the other eye serving as a control. Six rabbits are employed for each composition. Observations are made after 1, 24, 48, and 72 hours and 7 and 14 days after initial instillation. Results may vary from substan tially no change or only a slight irritation in the appearance of the rabbit's eye after 7 days to severe irritation and/or complete corneal opacity. Ocular lesions are scored for the cornea, iris and conjunctiva with a higher number indicating greater ocular irritation and the scores are added to give a total numerical value for each reading averaged from six rabbits. The averaged score is an indication of the irritation potential of the composition under test. Based on the averaged score, descriptive irritation evaluation may be given, e.g., none, slight, moderate or severe, as the case may be.

Test solutions were prepared and tested for eye irritation potential and for hair shampooing performance to determine the preferred combination for properties of mildness, "no sting", cleansing ability and aesthetics. Table I lists the weight percentage of the components present in Examples 1 through 7, followed by a summary of the Draize Test results of rabbit eye irritation for Examples 1 through 7. Examples 1 through 7, and all examples to follow, were prepared using mixing techniques well known in the shampoo art. The complete eye irritation data for Examples 1 through 7 can be found in Tables 1 through 7, respectively.

| Ingred. | EX. 1 | EX. 2 | EX. 3 | EX. 4 | EX. 5 | EX. 6 | EX. 7 |
|---|---|---|---|---|---|---|---|
| | % by weight | | | | | | |
| Soft Water | q.s. to 100% | | | | | | |
| Ammonium Nonoxynol-4 Sulfate | 4.05 | 4.05 | 4.05 | | 4.05 | | |
| PEG-120 Methyl Glucose Dioleate | 0.50 | 0.50 | 0.50 | 0.50 | 0.50 | 0.50 | 0.50 |
| Cocamido-propyl Betaine | | 2.10 | | 2.10 | 2.10 | 2.10 | 2.10 |
| Cocoampho-carboxy-glycinate | | | 6.00 | 6.00 | 6.00 | 6.00 | 6.00 |
| Ammonium Lauryl Sulfate | | | | | | 4.20 | |
| Sodium Trideceth Sulfate | | | | | | | 4.05 |
| Citric Acid | q.s. to pH 6.0 to 6.5 | | | | | | |

DRAIZE TEST - RABBIT EYE IRRITATION

| | EX. 1 | EX. 2 | EX. 3 | EX. 4 | EX. 5 | EX. 6 | EX. 7 |
|---|---|---|---|---|---|---|---|
| 1 hr. | 7.7 | 7.7 | 19.2 | 9.0 | 14.6 | 30.0 | 22.5 |
| 24 hr. | 2.3 | 3.7 | 19.5 | 4.0 | 11.7 | 19.7 | 17.9 |
| 48 hr. | 1.0 | 1.0 | 10.0 | 2.3 | 5.0 | 15.6 | 11.0 |
| 72 hr. | 0.0 | 0.0 | 3.8 | 0.7 | 2.1 | 10.5 | 7.7 |
| 7 days | 0.0 | 0.0 | 0.0 | 0.0 | 0.0 | 3.2 | 0.7 |
| 14 days | 0.0 | 0.0 | 0.0 | 0.0 | 0.0 | 4.2 | 0.0 |

TABLE I

RABBIT EYE IRRITATION RESULTS

CLIENT: HELENE CURTIS INDUSTRIES

Example: 1

MAXIMUM MEAN IRRITATION SCORE: 7.7/110.0

FORM ADMINISTERED: 0.1 ml instilled into the eye

SPECIAL INSTRUCTIONS: No Wash

| Tissue | Rabbit Number | 1 Hour | 24 Hours | 48 Hours | 72 Hours | 7 Days | 14 Days |
|---|---|---|---|---|---|---|---|
| Cornea (D-A) | 4 | 0 | 0 | 0 | 0 | 0 | 0 |
| Iris | | 0 | 0 | 0 | 0 | 0 | 0 |
| Conjunctiva (R-S-D) | | 6 (1-1-1) | 4 (1-1-0) | 2 (1-0-0) | 0 | 0 | 0 |
| Total | | 6 | 4 | 2 | 0 | 0 | 0 |
| Cornea (D-A) | 5 | 0 | 0 | 0 | 0 | 0 | 0 |
| Iris | | 0 | 0 | 0 | 0 | 0 | 0 |
| Conjunctiva (R-S-D) | | 6 (1-1-1) | 0 | 0 | 0 | 0 | 0 |
| Total | | 6 | 0 | 0 | 0 | 0 | 0 |
| Cornea (D-A) | 6 | 0 | 0 | 0 | 0 | 0 | 0 |
| Iris | | 0 | 0 | 0 | 0 | 0 | 0 |
| Conjunctiva (R-S-D) | | 8 (2-1-1) | 2 (1-0-0) | 0 | 0 | 0 | 0 |
| Total | | 8 | 2 | 0 | 0 | 0 | 0 |
| **Averages:** | | | | | | | |
| Cornea | | 0 | 0 | 0 | 0 | 0 | 0 |
| Iris | | 0 | 0 | 0 | 0 | 0 | 0 |
| Conjunctiva | | 7.7 | 2.3 | 1.0 | 0 | 0 | 0 |
| Total | | 7.7 | 2.3 | 1.0 | 0 | 0 | 0 |

Cornea:

D = Density
A = Area
Corneal Score = D x A x 5
Maximum Score = 20

Iris:

Iris Score = Value x 5
Maximum Score = 10

Conjunctiva:

Redness = R
Swelling = S
Discharge = D
Conjunctival Score = (R+S+D) x 2
Maximum Score = 20

## TABLE II

### RABBIT EYE IRRITATION RESULTS

**CLIENT:** HELENE CURTIS INDUSTRIES

**Example:** 2

**MAXIMUM MEAN IRRITATION SCORE:** 7.7/110.0

**FORM ADMINISTERED:** 0.1 ml instilled into the eye

**SPECIAL INSTRUCTIONS:** No Wash

| Tissue | Rabbit Number | 1 Hour | 24 Hours | 48 Hours | 72 Hours | 7 Days | 14 Days |
|---|---|---|---|---|---|---|---|
| Cornea (D-A) | 4 | 0 | 0 | 0 | 0 | 0 | 0 |
| Iris | | 0 | 0 | 0 | 0 | 0 | 0 |
| Conjunctiva (R-S-D) | | 8 (2-1-1) | 8 (2-1-1) | 2 (1-0-0) | 0 | 0 | 0 |
| Total | | 8 | 8 | 2 | 0 | 0 | 0 |
| Cornea (D-A) | 5 | 0 | 0 | 0 | 0 | 0 | 0 |
| Iris | | 0 | 0 | 0 | 0 | 0 | 0 |
| Conjunctiva (R-S-D) | | 8 (2-1-1) | 2 (1-0-0) | 0 | 0 | 0 | 0 |
| Total | | 8 | 2 | 0 | 0 | 0 | 0 |
| Cornea (D-A) | 6 | 0 | 0 | 0 | 0 | 0 | 0 |
| Iris | | 0 | 0 | 0 | 0 | 0 | 0 |
| Conjunctiva (R-S-D) | | 12 (2-2-2) | 6 (1-1-1) | 2 (1-0-0) | 0 | 0 | 0 |
| Total | | 12 | 6 | 2 | 0 | 0 | 0 |
| **Averages:** | | | | | | | |
| Cornea | | 0 | 0 | 0 | 0 | 0 | 0 |
| Iris | | 0 | 0 | 0 | 0 | 0 | 0 |
| Conjunctiva | | 7.7 | 3.7 | 1.0 | 0 | 0 | 0 |
| Total | | 7.7 | 3.7 | 1.0 | 0 | 0 | 0 |

Cornea:

D = Density
A = Area
Corneal Score = D x A x 5
Maximum Score = 80

Iris:

Iris Score = Value x 5
Maximum Score = 10

Conjunctiva:

Redness = R
Swelling = S
Discharge = D
Conjunctival Score = (R+S+D) x 2
Maximum Score = 20

## TABLE III

### RABBIT EYE IRRITATION RESULTS

**CLIENT:** HELENE CURTIS INDUSTRIES

**Example:** 3 **MAXIMUM MEAN IRRITATION SCORE:** 14.6/110.0

**FORM ADMINISTERED:** 0.1 ml instilled into the eye

**SPECIAL INSTRUCTIONS:** No Wash

| Tissue | Rabbit Number | 1 Hour | 24 Hours | 48 Hours | 72 Hours | 7 Days | 14 Days |
|---|---|---|---|---|---|---|---|
| Cornea (D-A) | 4 | 5 (1-1) | 5 (1-1) | 0 | 0 | 0 | 0 |
| Iris | | 0 | 0 | 0 | 0 | 0 | 0 |
| Conjunctiva (R-S-D) | | 12 (2-2-2) | 8 (2-1-1) | 4 (1-0-1) | 2 (1-0-0) | 0 | 0 |
| Total | | 17 | 13 | 4 | 2 | 0 | 0 |
| Cornea (D-A) | 5 | 10 (1-2) | 15 (1-3) | 10 (1-2) | 5 (1-1) | 0 | 0 |
| Iris | | 0 | 0 | 0 | 0 | 0 | 0 |
| Conjunctiva (R-S-D) | | 12 (2-2-2) | 12 (2-2-2) | 8 (2-1-1) | 4 (1-0-1) | 0 | 0 |
| Total | | 22 | 27 | 18 | 9 | 0 | 0 |
| Cornea (D-A) | 6 | 0 | 5 (1-1) | 0 | 0 | 0 | 0 |
| Iris | | 0 | 0 | 0 | 0 | 0 | 0 |
| Conjunctiva (R-S-D) | | 8 (2-1-1) | 8 (2-1-1) | 4 (1-0-1) | 2 (1-0-0) | 0 | 0 |
| Total | | 8 | 13 | 4 | 2 | 0 | 0 |
| **Averages:** | | | | | | | |
| Cornea | | 3.3 | 5.0 | 1.7 | 0.8 | 0 | 0 |
| Iris | | 0.0 | 0.0 | 0.0 | 0.0 | 0 | 0 |
| Conjunctiva | | 11.3 | 6.7 | 3.3 | 1.3 | 0 | 0 |
| Total | | 14.6 | 11.7 | 5.0 | 2.1 | 0 | 0 |

Cornea:

D = Density
A = Area
Corneal Score = D x A x 5
Maximum Score = 80

Iris:

Iris Score = Value x 5
Maximum Score = 10

Conjunctiva:

Redness = R
Swelling = S
Discharge = D
Conjunctival Score = (R+S+D) x 2
Maximum Score = 20

# TABLE IV

## RABBIT EYE IRRITATION RESULTS

**CLIENT:** HELENE CURTIS INDUSTRIES

**Example:** 4 **MAXIMUM MEAN IRRITATION SCORE:** 30.0/110.0

**FORM ADMINISTERED:** 0.1 ml instilled into the eye

**SPECIAL INSTRUCTIONS:** No Wash

| Tissue | Rabbit Number | 1 Hour | 24 Hours | 48 Hours | 72 Hours | 7 Days | 14 Days |
|---|---|---|---|---|---|---|---|
| Cornea (D-A) | 4 | 15 (1-3) | 15 (1-3) | 10 (1-2) | 10 (1-2) | 10 (1-2) | 10 (2-1) |
| Iris | | 0 | 0 | 5 | 5 | 0 | 5 |
| Conjunctiva (R-S-D) | | 10 (2-2-1) | 10 (2-2-1) | 8 (2-1-1) | 8 (2-1-1) | 6 (1-1-1) | 10 (2-1-2) |
| Total | | 25 | 25 | 23 | 23 | 16 | 25 |
| Cornea (D-A) | 5 | 20 (1-4) | 20 (1-4) | 15 (1-3) | 10 (1-2) | 0 | 0 |
| Iris | | 0 | 0 | 0 | 0 | 0 | 0 |
| Conjunctiva (R-S-D) | | 16 (2-3-3) | 12 (2-2-2) | 12 (2-2-2) | 10 (2-1-2) | 2 (1-0-0) | 0 |
| Total | | 36 | 32 | 27 | 20 | 2 | 0 |
| Cornea (D-A) | 6 | 10 (1-2) | 15 (1-3) | 10 (1-2) | 5 (1-1) | 0 | 0 |
| Iris | | 0 | 0 | 0 | 0 | 0 | 0 |
| Conjunctiva (R-S-D) | | 12 (2-2-2) | 10 (2-2-1) | 8 (2-1-1) | 4 (1-0-1) | 0 | 0 |
| Total | | 22 | 25 | 18 | 9 | 0 | 0 |
| **Averages:** | | | | | | | |
| Cornea | | 18.3 | 11.7 | 7.5 | 5.0 | 1.7 | 1.7 |
| Iris | | 0.0 | 0.0 | 0.8 | 0.8 | 0.0 | 0.8 |
| Conjunctiva | | 11.7 | 8.0 | 7.3 | 4.7 | 1.3 | 1.7 |
| Total | | 30.0 | 19.7 | 15.6 | 10.5 | 3.0 | 4.2 |

Cornea:

D = Density
A = Area
Corneal Score = D x A x 5
Maximum Score = 80

Iris:

Iris Score = Value x 5
Maximum Score = 10

Conjunctiva:

Redness = R
Swelling = S
Discharge = D
Conjunctival Score = (R+S+D) x 2
Maximum Score = 20

## TABLE V

### RABBIT EYE IRRITATION RESULTS

**CLIENT:** HELENE CURTIS INDUSTRIES

**Example:** 5

**MAXIMUM MEAN IRRITATION SCORE:** 22.5/110.0

**FORM ADMINISTERED:** 0.1 ml instilled into the eye

**SPECIAL INSTRUCTIONS:** No Wash

| Tissue | Rabbit Number | 1 Hour | 24 Hours | 48 Hours | 72 Hours | 7 Days | 14 Days |
|---|---|---|---|---|---|---|---|
| Cornea (D-A) | 4 | 5 (1-1) | 10 (1-2) | 5 (1-1) | 5 (1-1) | 0 | 0 |
| Iris | | 0 | 0 | 0 | 0 | 0 | 0 |
| Conjunctiva (R-S-D) | | 12 (2-2-2) | 6 (1-1-1) | 4 (1-0-1) | 2 (1-0-0) | 0 | 0 |
| Total | | 17 | 16 | 9 | 7 | 0 | 0 |
| Cornea (D-A) | 5 | 20 (1-4) | 15 (1-3) | 10 (1-2) | 10 (1-2) | 0 | 0 |
| Iris | | 0 | 0 | 0 | 0 | 0 | 0 |
| Conjunctiva (R-S-D) | | 12 (2-2-2) | 12 (2-2-2) | 8 (2-1-1) | 6 (2-0-1) | 2 (1-0-0) | 0 |
| Total | | 32 | 27 | 18 | 16 | 2 | 0 |
| Cornea (D-A) | 6 | 10 (1-2) | 5 (1-1) | 5 (1-1) | 0 | 0 | 0 |
| Iris | | 0 | 0 | 0 | 0 | 0 | 0 |
| Conjunctiva (R-S-D) | | 12 (2-2-2) | 10 (2-2-1) | 8 (2-1-1) | 4 (1-0-1) | 0 | 0 |
| Total | | 22 | 15 | 13 | 4 | 0 | 0 |
| **Averages:** | | | | | | | |
| Cornea | | 10.8 | 9.2 | 5.0 | 4.2 | 0 | 0 |
| Iris | | 0.0 | 0.0 | 0.0 | 0.0 | 0 | 0 |
| Conjunctiva | | 11.7 | 8.7 | 6.0 | 3.7 | 0.7 | 0 |
| Total | | 22.5 | 17.9 | 11.0 | 7.7 | 0.7 | 0 |

Cornea:

D = Density
A = Area
Corneal Score = D x A x 5
Maximum Score = 80

Iris:

Iris Score = Value x 5
Maximum Score = 10

Conjunctiva:

Redness = R
Swelling = S
Discharge = D
Conjunctival Score = (R+S+D) x 2
Maximum Score = 20

## TABLE VI

### RABBIT EYE IRRITATION RESULTS

CLIENT: HELENE CURTIS INDUSTRIES

Example: 6

MAXIMUM MEAN IRRITATION SCORE: 9.0/110.0

FORM ADMINISTERED: 0.1 ml instilled into the eye

SPECIAL INSTRUCTIONS: No Wash

| Tissue | Rabbit Number | 1 Hour | 24 Hours | 48 Hours | 72 Hours | 7 Days | 14 Days |
|---|---|---|---|---|---|---|---|
| Cornea (D-A) | 4 | 0 | 0 | 0 | 0 | 0 | 0 |
| Iris | | 0 | 0 | 0 | 0 | 0 | 0 |
| Conjunctiva (R-S-D) | | 8 (2-1-1) | 4 (1-1-0) | 2 (1-0-0) | 2 (1-0-0) | 0 | 0 |
| Total | | 8 | 4 | 2 | 2 | 0 | 0 |
| Cornea (D-A) | 5 | 0 | 0 | 0 | 0 | 0 | 0 |
| Iris | | 0 | 0 | 0 | 0 | 0 | 0 |
| Conjunctiva (R-S-D) | | 8 (2-1-1) | 2 (1-0-0) | 2 (1-0-0) | 0 | 0 | 0 |
| Total | | 8 | 2 | 2 | 0 | 0 | 0 |
| Cornea (D-A) | 6 | 0 | 0 | 0 | 0 | 0 | 0 |
| Iris | | 0 | 0 | 0 | 0 | 0 | 0 |
| Conjunctiva (R-S-D) | | 8 (2-1-1) | 2 (1-0-0) | 2 (1-0-0) | 0 | 0 | 0 |
| Total | | 8 | 2 | 2 | 0 | 0 | 0 |
| **Averages:** | | | | | | | |
| Cornea | | 0 | 0 | 0 | 0 | 0 | 0 |
| Iris | | 0 | 0 | 0 | 0 | 0 | 0 |
| Conjunctiva | | 9.0 | 4.0 | 2.3 | 0.7 | 0 | 0 |
| Total | | 9.0 | 4.0 | 2.3 | 0.7 | 0 | 0 |

Cornea:

D = Density
A = Area
Corneal Score = D x A x 5
Maximum Score = 80

Iris:

Iris Score = Value x 5
Maximum Score = 10

Conjunctiva:

Redness = R
Swelling = S
Discharge = D
Conjunctival Score = (R+S+D) x 2
Maximum Score = 20

## TABLE VII
### RABBIT EYE IRRITATION RESULTS

**CLIENT:** HELENE CURTIS INDUSTRIES

**Example:** 7

**MAXIMUM MEAN IRRITATION SCORE:** 19.5/110.0

**FORM ADMINISTERED:** 0.1 ml installed into the eye

**SPECIAL INSTRUCTIONS:** No Wash

| Tissue | Rabbit Number | 1 Hour | 24 Hours | 48 Hours | 72 Hours | 7 Days | 14 Days |
|---|---|---|---|---|---|---|---|
| Cornea (D-A) | 4 | 10 (1-2) | 5 (1-1) | 0 | 0 | 0 | 0 |
| Iris | | 0 | 0 | 0 | 0 | 0 | 0 |
| Conjunctiva (R-S-D) | | 12 (2-2-2) | 10 (2-2-1) | 8 (2-1-1) | 4 (1-0-1) | 0 | 0 |
| Total | | 22 | 15 | 8 | 4 | 0 | 0 |
| Cornea (D-A) | 5 | 5 (1-1) | 5 (1-1) | 0 | 0 | 0 | 0 |
| Iris | | 0 | 0 | 0 | 0 | 0 | 0 |
| Conjunctiva (R-S-D) | | 8 (2-1-1) | 8 (2-1-1) | 4 (1-0-1) | 2 (1-0-0) | 0 | 0 |
| Total | | 13 | 13 | 4 | 2 | 0 | 0 |
| Cornea (D-A) | 6 | 10 (1-2) | 10 (1-2) | 5 (1-1) | 0 | 0 | 0 |
| Iris | | 0 | 0 | 0 | 0 | 0 | 0 |
| Conjunctiva (R-S-D) | | 10 (2-2-1) | 12 (2-2-2) | 8 (2-1-1) | 4 (1-0-1) | 0 | 0 |
| Total | | 20 | 22 | 13 | 4 | 0 | 0 |
| **Averages:** | | | | | | | |
| Cornea | | 9.2 | 9.2 | 3.3 | 0.8 | 0 | 0 |
| Iris | | 0.0 | 0.0 | 0.0 | 0.0 | 0 | 0 |
| Conjunctiva | | 10.0 | 10.3 | 6.7 | 3.0 | 0 | 0 |
| Total | | 19.2 | 19.5 | 10.0 | 3.8 | 0 | 0 |

Cornea:

D = Density
A = Area
Corneal Score = D x A x 5
Maximum Score = 80

Iris:

Iris Score = Value x 5
Maximum Score = 10

Conjunctiva:

Redness = R
Swelling = S
Discharge = D
Conjunctival Score = (R+S+D) x 2
Maximum Score = 20

Example 1 is a pH-adjusted aqueous solution of ammonium nonoxynol-4 sulfate and a glucose ester, namely PEG-120 methyl glucose dioleate, as a viscosity enhancer and conditioner. In Example 2, the addition of cocamidopropyl betaine did not increase the eye irritation level, although the total surfactant concentration has increased. Example 3 shows increased eye irritation when cocoamphocarboxyglycinate replaces the betaine, however, reintroduction of the betaine in Example 5 shows a definite improvement in eye irritancy over Example 3. Example 4 shows the extent of eye irritation in shampoos lacking an anionic surfactant, and although the eye irritation levels are good, absence of an anionic surfactant severely retards the cleaning and foaming ability of the shampoo. The increased irritation levels of Examples 3 and 5 over Examples 1, 2 and 4 also is due in part to greater surfactant levels in Examples 3 and 5. For comparison, in Example 6 ammonium lauryl sulfate replaced the ammonium nonoxynol-4 sulfate of Example 5 on a weight basis and exhibited a much higher eye irritation level of 30.0, or a 105.5% increase over the eye irritancy level of 14.6 for the composition of Example 5.

A composition of the present invention (Example 5) is compared to a composition containing an anionic surfactant, sodium trideceth sulfate, used in a leading commercial baby shampoo (Example 7). The resultant eye irritation tests showed a 54% increase for the composition of Example 7 containing sodium trideceth sulfate over the eye irritation levels of Example 5, containing ammonium nonoxynol-4 sulfate.

The compositions of examples 8 through 12 were prepared to demonstrate the low irritancy and improved viscosities of several compositions of the present invention. As will be more fully described hereinafter, the viscosities of the compositions of Examples 8 through 12 are all substantially improved over the leading commercial baby shampoos; and eye irritancy levels of the compositions of Examples 8 through 12 are less than or equal to the commercial shampoos. The compositions of Examples 8 through 12 also exhibit improved cleansing and aesthetic properties.

| Ingred. (present by wt. %) | EX. 8 | EX. 9 | EX. 10 | EX. 11 | EX 12 |
|---|---|---|---|---|---|
| Water | -----------q.s. to 100%----------- | | | | |
| Cocoampho-carboxyglycinate | 5.00 | 5.00 | 5.00 | 5.95 | 5.95 |
| Ammonium non-oxynol-4 sulfate | 2.70 | 2.70 | 2.70 | 5.40 | 4.05 |
| Cocamidopropyl hydroxy-sultaine | 1.50 | --- | 2.45 | --- | --- |
| Cocamidopropyl betaine | --- | 1.35 | --- | --- | 2.10 |
| Alkyl glucoside | 1.40 | 1.40 | 1.40 | --- | --- |
| PEG-120 Methyl glucose dioleate | 1.50 | 1.50 | 1.00 | 1.00 | 0.50 |
| PEG-80 Sorbitan laurate | --- | --- | --- | 1.50 | 2.50 |
| Citric acid | ------q.s. to pH 6.0 to 6.5------- | | | | |
| Color, fragrance preservative | ---------------q.s.--------------- | | | | |
| Viscosity | 3100 | 2500 | 2000 | 5400 | 2540 |
| Draize Results | | | | | |
| 1 hr. | 7.7 | 8.0 | 7.3 | 12.7 | 14.0 |
| 24 hr. | 3.0 | 4.3 | 5.7 | 7.3 | 10.7 |
| 48 hr. | 1.3 | 1.0 | 2.7 | 4.7 | 5.4 |
| 72 hr. | 0.0 | 0.0 | 0.7 | 1.3 | 1.3 |

In comparing the compositions of Example 8 and Example 9 it is apparent that substituting cocamidopropyl betaine for a cocamidopropyl hydroxysultaine does not appreciably alter the eye irritation level, but it does lead to a decrease in viscosity. In the composition of Example 10, increased cocamidopropyl hydroxysultaine levels results in very slightly improved eye irritation potential, and a decreased PEG-120 methyl glucose dioleate level leads to a viscosity decrease. In the composition of Example 11, omitting the betaine-type of surfactant and increasing the amount of cocoamphocarboxyglycinate and ammonium non-oxynol-4 sulfate leads to increased eye irritation potential, as expected from doubling the anionic surfactant level and omitting the mild betaine-type surfactants. The effects of

incorporating the PEG-120 methyl glucose dioleate into compositions of the present invention are shown in Example 12. Lower viscosities and increased eye irritation levels resulted from reducing the glucose dioleate concentration and increasing the amount of PEG-80 sorbitan laurate even though a betaine was reintroduced into the composition and the anionic surfactant level was decreased. The substantially different nature of the decreased eye irritation mechanism for the compositions of the present invention is further emphasized by the fact that increased quantities of the nonionic PEG-80 sorbitan laurate does not decrease eye irritation levels, as it does in commercial baby shampoos. To achieve the full advantage of the present invention, PEG-120 methyl glucose dioleate is included at low levels of about 1% to about 2% by weight of the total composition as a nonionic viscosity enhancer. At this level, the glucose oleate effectively thickens the shampoo composition without adversely affecting foam properties, and minimizes the irritation levels of the anionic/amphoteric blend. Tables VIII through XII contain the complete eye irritation data for Examples 8 through 12.

TABLE VIII

**RABBIT EYE IRRITATION RESULTS**

**CLIENT:** HELENE CURTIS INDUSTRIES

**Example:** 8

**MAXIMUM MEAN IRRITATION SCORE: 7.7/110.0**

**FORM ADMINISTERED:** 0.1 ml instilled into the eye

**SPECIAL INSTRUCTIONS:** No Wash

| Tissue | Rabbit Number | 1 Hour | 24 Hours | 48 Hours | 72 Hours | 7 Days | 14 Days |
|---|---|---|---|---|---|---|---|
| Cornea (D-A) | 4 | 0 | 0 | 0 | 0 | 0 | 0 |
| Iris | | 0 | 0 | 0 | 0 | 0 | 0 |
| Conjunctiva (R-S-D) | | 6 (1-1-1) | 2 (1-0-0) | 0 | 0 | 0 | 0 |
| Total | | 6 | 2 | 0 | 0 | 0 | 0 |
| Cornea (D-A) | 5 | 0 | 0 | 0 | 0 | 0 | 0 |
| Iris | | 0 | 0 | 0 | 0 | 0 | 0 |
| Conjunctiva (R-S-D) | | 8 (1-1-2) | 4 (1-1-0) | 2 (1-0-0) | 0 | 0 | 0 |
| Total | | 8 | 4 | 2 | 0 | 0 | 0 |
| Cornea (D-A) | 6 | 0 | 0 | 0 | 0 | 0 | 0 |
| Iris | | 0 | 0 | 0 | 0 | 0 | 0 |
| Conjunctiva (R-S-D) | | 8 (1-1-2) | 4 (1-0-1) | 2 (1-0-0) | 0 | 0 | 0 |
| Total | | 8 | 4 | 2 | 0 | 0 | 0 |
| **Averages:** | | | | | | | |
| Cornea | | 0 | 0 | 0 | 0 | 0 | 0 |
| Iris | | 0 | 0 | 0 | 0 | 0 | 0 |
| Conjunctiva | | 7.7 | 3.0 | 1.3 | 0 | 0 | 0 |
| Total | | 7.7 | 3.0 | 1.3 | 0 | 0 | 0 |

Cornea:

D = Density
A = Area
Corneal Score = D x A x 5
Maximum Score = 80

Iris:

Iris Score = Value x 5
Maximum Score = 10

Conjunctiva:

Redness = R
Swelling = S
Discharge = D
Conjunctival Score = (R+S+D) x 2
Maximum Score = 20

## TABLE IX

### RABBIT EYE IRRITATION RESULTS

**CLIENT:** HELENE CURTIS INDUSTRIES

**Example:** 9

**MAXIMUM MEAN IRRITATION SCORE:** 8.0/110.0

**FORM ADMINISTERED:** 0.1 ml instilled into the eye

**SPECIAL INSTRUCTIONS:** No Wash

| Tissue | Rabbit Number | 1 Hour | 24 Hours | 48 Hours | 72 Hours | 7 Days | 14 Days |
|---|---|---|---|---|---|---|---|
| Cornea (D-A) | 4 | 0 | 0 | 0 | 0 | 0 | 0 |
| Iris | | 0 | 0 | 0 | 0 | 0 | 0 |
| Conjunctiva (R-S-D) | | 6 (1-1-1) | 2 (1-0-0) | 0 | 0 | 0 | 0 |
| Total | | 6 | 2 | 0 | 0 | 0 | 0 |
| Cornea (D-A) | 5 | 0 | 0 | 0 | 0 | 0 | 0 |
| Iris | | 0 | 0 | 0 | 0 | 0 | 0 |
| Conjunctiva (R-S-D) | | 8 (1-1-2) | 2 (1-0-0) | 0 | 0 | 0 | 0 |
| Total | | 8 | 2 | 0 | 0 | 0 | 0 |
| Cornea (D-A) | 6 | 0 | 0 | 0 | 0 | 0 | 0 |
| Iris | | 0 | 0 | 0 | 0 | 0 | 0 |
| Conjunctiva (R-S-D) | | 8 (1-1-2) | 6 (1-1-1) | 2 (1-0-0) | 0 | 0 | 0 |
| Total | | 8 | 6 | 2 | 0 | 0 | 0 |
| **Averages:** | | | | | | | |
| Cornea | | 0 | 0 | 0 | 0 | 0 | 0 |
| Iris | | 0 | 0 | 0 | 0 | 0 | 0 |
| Conjunctiva | | 8.0 | 4.3 | 1.0 | 0 | 0 | 0 |
| Total | | 8.0 | 4.3 | 1.0 | 0 | 0 | 0 |

Cornea:
D = Density
A = Area
Corneal Score = D x A x 5
Maximum Score = 80

Iris:
Iris Score = Value x 5
Maximum Score = 10

Conjunctiva:
Redness = R
Swelling = S
Discharge = D
Conjunctival Score = (R+S+D) x 2
Maximum Score = 20

TABLE X

**RABBIT EYE IRRITATION RESULTS**

**CLIENT:** HELENE CURTIS INDUSTRIES

**Example:** 10 **MAXIMUM MEAN IRRITATION SCORE:** 7.3/110.0

**FORM ADMINISTERED:** 0.1 ml instilled into the eye

**SPECIAL INSTRUCTIONS:** No Wash

| Tissue | Rabbit Number | 1 Hour | 24 Hours | 48 Hours | 72 Hours | 7 Days | 14 Days |
|---|---|---|---|---|---|---|---|
| Cornea (D-A) | 4 | 0 | 0 | 0 | 0 | 0 | 0 |
| Iris | | 0 | 0 | 0 | 0 | 0 | 0 |
| Conjunctiva (R-S-D) | | 8 (1-1-2) | 6 (1-1-1) | 4 (1-0-1) | 0 | 0 | 0 |
| Total | | 8 | 6 | 4 | 0 | 0 | 0 |
| Cornea (D-A) | 5 | 0 | 0 | 0 | 0 | 0 | 0 |
| Iris | | 0 | 0 | 0 | 0 | 0 | 0 |
| Conjunctiva (R-S-D) | | 6 (1-1-1) | 6 (1-1-1) | 4 (1-0-1) | 2 (1-0-0) | 0 | 0 |
| Total | | 6 | 6 | 4 | 2 | 0 | 0 |
| Cornea (D-A) | 6 | 0 | 0 | 0 | 0 | 0 | 0 |
| Iris | | 0 | 0 | 0 | 0 | 0 | 0 |
| Conjunctiva (R-S-D) | | 8 (1-1-2) | 6 (1-1-1) | 2 (1-0-0) | 2 (1-0-0) | 0 | 0 |
| Total | | 8 | 6 | 2 | 2 | 0 | 0 |
| **Averages:** | | | | | | | |
| Cornea | | 0 | 0 | 0 | 0 | 0 | 0 |
| Iris | | 0 | 0 | 0 | 0 | 0 | 0 |
| Conjunctiva | | 7.3 | 5.7 | 2.7 | 0.7 | 0 | 0 |
| Total | | 7.3 | 5.7 | 2.7 | 0.7 | 0 | 0 |

Cornea:

D = Density
A = Area
Corneal Score = D x A x 5
Maximum Score = 80

Iris:

Iris Score = Value x 5
Maximum Score = 10

Conjunctiva:

Redness = R
Swelling = S
Discharge = D
Conjunctival Score = (R+S+D) x 2
Maximum Score = 20

TABLE XI

RABBIT EYE IRRITATION RESULTS

CLIENT: HELENE CURTIS INDUSTRIES

Example: 11

MAXIMUM MEAN IRRITATION SCORE: 12.7/110.0

FORM ADMINISTERED: 0.1 ml instilled into the eye

CLASSIFICATION: ----

SPECIAL INSTRUCTIONS: No Wash

| Tissue | Rabbit Number | 1 Hour | 24 Hours | 48 Hours | 72 Hours | 7 Days | 14 Days |
|---|---|---|---|---|---|---|---|
| Cornea (D-A) | 1 | 0 | 0 | 0 | 0 | 0 | 0 |
| Iris | | 0 | 0 | 0 | 0 | 0 | 0 |
| Conjunctiva (R-S-D) | | 12 (2-2-2) | 8 (2-1-1) | 6 (1-1-1) | 2 (1-0-0) | 0 | 0 |
| Total | | 12 | 8 | 6 | 2 | 0 | 0 |
| Cornea (D-A) | 2 | 0 | 0 | 0 | 0 | 0 | 0 |
| Iris | | 0 | 0 | 0 | 0 | 0 | 0 |
| Conjunctiva (R-S-D) | | 14 (2-2-3) | 6 (1-1-1) | 4 (1-0-1) | 2 (1-0-0) | 0 | 0 |
| Total | | 14 | 6 | 4 | 2 | 0 | 0 |
| Cornea (D-A) | 3 | 0 | 0 | 0 | 0 | 0 | 0 |
| Iris | | 0 | 0 | 0 | 0 | 0 | 0 |
| Conjunctiva (R-S-D) | | 12 (1-2-3) | 8 (1-1-2) | 4 (1-0-1) | 0 | 0 | 0 |
| Total | | 12 | 8 | 4 | 0 | 0 | 0 |
| **Averages:** | | | | | | | |
| Cornea | | 0 | 0 | 0 | 0 | 0 | 0 |
| Iris | | 0 | 0 | 0 | 0 | 0 | 0 |
| Conjunctiva | | 12.7 | 7.3 | 4.7 | 1.3 | 0 | 0 |
| Total | | 12.7 | 7.3 | 4.7 | 1.3 | 0 | 0 |

Cornea:

D = Density
A = Area
Corneal Score = D x A x 5
Maximum Score = 80

Iris:

Iris Score = Value x 5
Maximum Score = 10

Conjunctiva:

Redness = R
Swelling = S
Discharge = D
Conjunctival Score = (R+S+D) x 2
Maximum Score = 20

## TABLE XII

## RABBIT EYE IRRITATION RESULTS

**CLIENT:** HELENE CURTIS INDUSTRIES

**Example:** 12

**MAXIMUM MEAN IRRITATION SCORE:** 14.0/110.0

**FORM ADMINISTERED:** 0.1 ml instilled into the eye

**SPECIAL INSTRUCTIONS:** No Wash

| Tissue | Rabbit Number | 1 Hour | 24 Hours | 48 Hours | 72 Hours | 7 Days | 14 Days |
|---|---|---|---|---|---|---|---|
| Cornea (D-A) | 4 | 0 | 0 | 0 | 0 | 0 | 0 |
| Iris | | 0 | 0 | 0 | 0 | 0 | 0 |
| Conjunctiva (R-S-D) | | 12 (2-2-2) | 10 (2-1-2) | 4 (1-0-1) | 2 (1-0-0) | 0 | 0 |
| Total | | 12 | 10 | 4 | 2 | 0 | 0 |
| Cornea (D-A) | 5 | 0 | 0 | 0 | 0 | 0 | 0 |
| Iris | | 0 | 0 | 0 | 0 | 0 | 0 |
| Conjunctiva (R-S-D) | | 8 (1-1-2) | 4 (1-0-1) | 2 (1-0-0) | 0 | 0 | 0 |
| Total | | 8 | 4 | 2 | 0 | 0 | 0 |
| Cornea (D-A) | 6 | 10 (1-2) | 10 (1-2) | 5 (1-1) | 0 | 0 | 0 |
| Iris | | 0 | 0 | 0 | 0 | 0 | 0 |
| Conjunctiva (R-S-D) | | 12 (2-2-2) | 12 (2-2-2) | 6 (1-1-1) | 2 (1-0-0) | 0 | 0 |
| Total | | 22 | 22 | 11 | 2 | 0 | 0 |
| **Averages:** | | | | | | | |
| Cornea | | 3.3 | 3.3 | 1.7 | 0 | 0 | 0 |
| Iris | | 0.0 | 0.0 | 0.0 | 0 | 0 | 0 |
| Conjunctiva | | 10.7 | 7.0 | 3.7 | 1.3 | 0 | 0 |
| Total | | 14.0 | 10.7 | 5.4 | 1.3 | 0 | 0 |

Cornea:

D = Density
A = Area
Corneal Score = D x A x 5
Maximum Score = 80

Iris:

Iris Score = Value x 5
Maximum Score = 10

Conjunctiva:

Redness = R
Swelling = S
Discharge = D
Conjunctival Score = (R+S+D) x 2
Maximum Score = 20

TABLE XIII

COMMERCIAL PRODUCT ANALYSIS AND COMPARISON

| COMMERCIAL PRODUCT | 1 HR. | 24 HRS. | 48 HRS. | 72 HRS. |
|---|---|---|---|---|
| A | 8.0 | 4.0 | 1.3 | 0.0 |
| B | 9.3 | 4.7 | 2.0 | 0.0 |
| C | 7.3 | 2.7 | 0.7 | 0.0 |
| D | 11.3 | 3.3 | 0.7 | 0.0 |
| E | 6.0 | 2.7 | 1.3 | 0.0 |
| F | 26.7 | 21.3 | 9.0 | 1.3 |
| EXAMPLE 8 | 7.7 | 3.0 | 1.3 | 0.0 |
| EXAMPLE 9 | 8.0 | 4.3 | 1.0 | 0.0 |
| EXAMPLE 10 | 7.3 | 5.7 | 2.7 | 0.7 |

As is apparent from the data in Table XIII, Examples 8 through 10 favorably compare with, or improve upon, the eye irritation levels of present commercial baby hair shampoos. However, in addition to comparable or improved irritation potential, the shampoos of the present invention unexpectedly retain the aesthetic and cleaning properties of the harsher, conventional alkyl sulfate-based hair shampoos.

The viscosity of a liquid detergent composition is extremely important for consumer appeal. The composition must be thin enough for dispensing, yet thick enough so that it does not immediately run through the user's fingers. A thick product is also perceived by the consumer to be concentrated and, therefore, an effective and economical product. Ideally, hair shampoos, except for the gelled tube-type compositions, have a viscosity in the range of about 2000 cps to about 8000 cps for easy dispensing, minimal runniness and good consumer appeal. From Table IV, it is obvious that the viscosity of present day baby shampoos is usually below 1500 cps, or well below the minimum viscosity targeted for good consumer acceptance. The viscosity of most shampoos and detergents may be increased by adding inorganic thickeners, such as ammonium or sodium chloride, or organic thicken ers, such as gums or nonionic surfactants; however, these additives generally increase the irritation and stinging properties of the shampoo. Hair shampoo compositions of the present invention are readily prepared in the 2000 cps to 8000 cps, and particularly in the 2000 cps to 5000 cps viscosity range, without the addition of inorganic thickeners, gums, or large amounts of nonionic surfactants, as shown in Table XIV.

TABLE XIV

PHYSICAL PROPERTIES OF BABY SHAMPOOS

| COMMERCIAL PRODUCT | pH | VISCOSITY (CPS) |
|---|---|---|
| J & J NO MORE TEARS | 6.15 | 1000 |
| MENNEN BABY MAGIC | 7.09 | 540 |
| VASELINE INTENSIVE CARE | 6.56 | 340 |
| WALGREENS | 6.43 | 1130 |
| OSCO | 6.16 | 480 |
| SUAVE | 7.50 | 650 |
| EXAMPLE 8 | 6.35 | 3100 |
| EXAMPLE 9 | 6.11 | 2500 |
| EXAMPLE 10 | 6.49 | 2000 |

Compositions of the present invention were clinically tested against the leading commercial baby shampoos by washing one-half of a subject's hair with a shampoo composition of the present invention and the other half of the subject's hair with the commercial shampoo. In this blind test, the operator rates each shampoo in several categories and issues comments as to why one shampoo is preferred over the other. In one clinical test, the shampoo composition of Example 8 was rated as performing better on the first lather, by being easier to lather up and with a better lathering ability and amount of foam when compared to the leading commercial baby shampoos. For the other testing parameters, such as combing, feel, static, effect on color, and the like, the two shampoos of this test were rated essentially equal. The shampoo composition of Example 10 provided the same benefits as Example 8, when compared to the same commercial baby shampoo, plus imparting improved latherability and amount of foam on the second lathering. In another test, the composition of Example 9 demonstrated a pronounced improvement in ease of application, probably due to its greater viscosity and increased anionic level; Example 9 also demonstrated improved lathering and foam generation ability. The composition of Example 13 was similarly tested against the same commercial shampoo, and showed improved lathering, foam generation and combing ability.

| Example 13 | |
|---|---|
| Water | q.s. to 100% |
| Cocoamphocarboxyglycinate | 5.95% |
| Ammonium nonoxynol-4 sulfate | 2.70% |
| Cocamidopropyl hydroxysultaine | 1.50% |
| Alkyl glucoside | 1.40% |
| PEG-120 methyl glucose dioleate | 0.50% |
| Citric acid | 0.35% |
| Dye/fragrance/preservative | q.s. |

pH - 6.32

viscosity - 1040 cps

From the Draize Test data, the comparative performance data and the physical properties of the compositions of the present invention, new and unexpected improvements in baby hair shampoo compositions are realized by combining a sulfated alkylphenol ethoxylate, a carboxylated derivative of a fatty imidazoline and a zwitterionic betaine. The compositions of the present invention unexpectedly demonstrate a lower eye and skin irritation potential, better foaming characteristics and improved viscosities at a generally lower total surfactant level, without the addition of thickeners.

The compositions of the present invention are primarily useful in shampoo formulations where high foaming characterictis as well as low ocular and skin irritation potential are desired. They may also be used as liquid soaps and cleansers such as baby bath compositions, skin cleansers, mild shampoos for delicate skin, bubble bath compositions, as well as in compositions suitable for cleansing animals and inanimate objects.

The compositions of the present invention possess the exceptionally desirable qualities of mildness and "no sting", plus the ability to thoroughly cleanse the hair and scalp with the generation of copious amounts of stable foam. The compositions of the present invention also possess aesthetic qualities unmatched by present day non-irritating commercial shampoos.

## Claims

1. A mild detergent composition characterised in that it comprises about 2% to about 10% by weight, based on the total weight of the composition, of a water soluble anionic surfactant of the formula

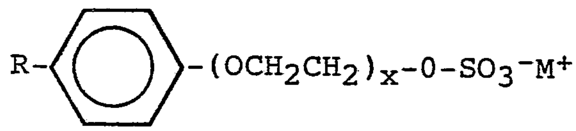

wherein R is an alkyl group containing about 6 to about 15 carbon atoms, x is an integer from 1 to about 10, and M is an alkali metal, ammonium, triethanolammonium, monoethanolammonium, diethanolammonium, or an alkylammonium containing 1 to 3 alkyl groups, each having 1 or 2 carbon atoms; about 3% to about 10% by weight, based on the total weight of the composition of an amphoteric, water soluble, carboxylated derivative of a fatty imidazoline; and about 1% to about 6% by weight, based on the total weight of the composition, of a water soluble betaine-type detergent of the formula

$$\begin{array}{c} R_2 \\ | \\ R_1-N^{+}-R_4-Y \\ | \\ R_3 \end{array}$$

wherein $R_1$ is an alkyl or acyl group containing about 8 to about 18 carbon atoms, $R_2$ and $R_3$, the same or different, are each an alkyl group having 1 to 4 carbon groups or 2-hydroxyethyl; Y is a carboxylate or sulfonate radical, and $R_4$ is a methylene group when Y is a carboxylate group or propylene or 2-hydroxypropylene when Y is a sulfonate group; in a suitable carrier.

2. A composition as claimed in claim 1, characterised in that the carboxylated derivative of a fatty imidazoline is an amphoteric detergent of the formula

$$\begin{array}{l} \qquad\qquad\qquad\qquad\quad CH_2CO_2^- \\ \qquad\qquad\qquad\qquad\quad | \\ R_5-CO-NH-C_2H_4-N^{+}-CH_2COOH \\ \qquad\qquad\qquad\qquad\quad | \\ \qquad\qquad\qquad\qquad\quad CH_2CH_2OH \end{array}$$

or

$$\begin{array}{l} \ \ O \qquad\qquad\qquad\qquad\quad CH_2CH_2OCH_2CH_2CO_2^-\ Na^+ \\ \ \ \| \qquad\qquad\qquad\qquad\quad\ | \\ R_6-C-NH-CH_2CH_2-N-CH_2CH_2CO_2^-\ Na^+ \end{array}$$

wherein $R_5$ and $R_6$, same or different, are each an alkyl group containing about 7 to about 17 carbon atoms.

3. A composition as claimed in either claim 1 or claim 2, characterised in that R is an alkyl group containing from about 7 to about 10 carbon atoms and in that x is a number from about 3 to about 7.

4. A composition as claimed in any one of the preceding claims, characterised in that the anionic surfactant is the ammonium salt of nonylphenoxypoly(ethyleneoxy)ethanol sulfate containing about 4 ethyleneoxy moieties per molecule, preferably present in an amount of about 2% to about 7% by weight, based on the total weight of the composition, and said composition has a pH of 5.5 to 7.5, preferably 6.0 to 7.0.

5. A composition as claimed in any one of the preceding claims, characterised in that the amphoteric detergent is cocoamphocarboxyglycinate or cocoamphocarboxypropionate.

6. A composition as claimed in any one of the preceding claims, characterised in that the amphoteric detergent is present in an amount of about 4% to about 7% by weight, based on the total weight of the composition.

7. A composition as claimed in any one of the preceding claims, characterised in that the betaine-type surfactant is a betaine, alkamidopropyl betaine, sulfobetaine or alkamidopropyl hydroxysultaine, preferably cocamidopropyl betaine or cocamidopropyl hydroxysultaine.

8. A composition as claimed in any one of the preceding claims, characterised in that the betaine-type surfactant is present in an amount of about 1% to about 4% by weight, based on the total weight of the composition.

9. A composition as claimed in any one of the preceding claims, characterised in that it further comprises from 0.1% to about 3% by weight, based on the entire weight of the composition, of a water soluble polyethoxylated mono-fatty acid ester or di-fatty acid ester of methyl glucose, wherein said fatty acid contains from about 8 to about 18 carbon atoms and said polyethoxylated mono-fatty acid ester or di-fatty acid ester of methyl glucose contains from about 80 to about 160 ethyleneoxy moieties per molecule.

10. A composition as claimed in claim 9, characterised in that the fatty acid or acids are ethylenically unsaturated fatty acids, preferably oleic acid.

11. A composition as claimed in either claim 9 or claim 10, characterised in that the methyl glucose ester is a di-fatty acid ester of methyl glucose, preferably methyl glucose dioleate containing about 120 ethoxy moieties per molecule.